(19) Europäisches Patentamt
European Patent Office
Office européen des brevets

(11) **EP 2 279 226 B1**

(12) **EUROPÄISCHE PATENTSCHRIFT**

(45) Veröffentlichungstag und Bekanntmachung des Hinweises auf die Patenterteilung:
**09.08.2017 Patentblatt 2017/32**

(51) Int Cl.:
*C09C 1/30* (2006.01)    *C08K 9/06* (2006.01)
*B82Y 30/00* (2011.01)    *C07C 51/44* (2006.01)

(21) Anmeldenummer: **09732109.5**

(22) Anmeldetag: **20.04.2009**

(86) Internationale Anmeldenummer:
**PCT/EP2009/002875**

(87) Internationale Veröffentlichungsnummer:
**WO 2009/127438 (22.10.2009 Gazette 2009/43)**

(54) **OBERFLÄCHENMODIFIZIERTE SILIZIUMDIOXID-PARTIKEL**

SURFACE MODIFIED SILICON DIOXIDE PARTICLES

PARTICULES DE DIOXYDE DE SILICIUM MODIFIÉES EN SURFACE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **18.04.2008 EP 08007625**

(43) Veröffentlichungstag der Anmeldung:
**02.02.2011 Patentblatt 2011/05**

(73) Patentinhaber: **Evonik Degussa GmbH**
**45128 Essen (DE)**

(72) Erfinder: **KUEHNER, Uwe, Dietrich**
**20457 Hamburg (DE)**

(56) Entgegenhaltungen:
EP-A- 0 982 268    US-A- 3 015 645
US-A- 5 942 590    US-A- 6 107 351
US-A1- 2003 138 715    US-A1- 2004 052 939
US-B1- 6 706 398

**Beschreibung**

Die vorliegende Erfindung betrifft modifizierte Siliziumdioxid-Partikel, sowie ein Verfahren zur Herstellung modifizierter Siliziumdioxid-Partikel und die durch dieses Verfahren erhältlichen Produkte, bestimmte Verwendungszwecke der Siliziumdioxid-Partikel und Nanocomposite, welche die Siliziumdioxid-Partikel enthalten. Ebenso werden Kieselsole enthaltend die modifizierten Siliziumdioxid-Partikel vorgeschlagen.

Kieselsol ist eine Suspension von amorphem Siliziumdioxid ($SiO_2$), in welcher das Siliziumdioxid in Form von im Wesentlichen (d.h. zu mindestens 50%, vorzugsweise zu mindestens 70, 80 oder 90%) untereinander unvernetzten, kugelförmigen Einzelpartikeln vorliegt. Das Dispersionsmittel kann verschieden sein, so. z.B. ein Lösungsmittel oder ein Monomer.

Kieselsole werden vielseitig eingesetzt. Beispielsweise sind sie für den Einsatz als Bindemittel für Feinguss, für Fasern im Feuerfestbereich und bei der Herstellung von Katalysatoren, als Beschichtungsagenzien für Folien (Antiblocking), im Textilsektor für Schiebefestausrüstungen, im Bausektor als Additive für Spritzbeton oder als Binder für Brand- und Wärmeschutzanwendungen, als Poliermittel für die Elektronik oder auch im Papiersektor, beispielsweise bei der Papierretention oder als Additiv in der Beschichtung von Spezialpapieren, geeignet.

Herkömmliche Kieselsole sind, je nach Teilchengröße der Siliziumdioxid-Partikel, milchig trüb über opaleszierend bis farblos klar ausgebildet. Die Partikel haben im Allgemeinen Durchmesser von 5 nm bis 150 nm und sind in der Regel kugelförmig, räumlich begrenzt und vorzugsweise elektrisch negativ geladen. Im Innern der einzelnen Partikel liegt üblicherweise ein Gerüst von Siloxanbindungen vor, welches sich aus der Verknüpfung von $[SiO_4]$-Tetraedem bzw. von Polykieselsäuren ergibt.

Aufgrund ihrer geringen Größe haben die Partikel eine große spezifische Oberfläche, was wiederum zu einer hohen Oberflächenenergie führt. Eine unerwünschte Folge dieser hohen Oberflächenenergie ist, dass die Partikel dazu neigen, Agglomerate oder sogar Aggregate zu bilden. Damit ist die Bildung stabiler, Siliziumdioxid-Partikel enthaltender Dispersionen nicht ohne weiteres möglich.

Um die Agglomeration oder die Aggregation der Partikel möglichst zu verhindern, ist aus dem Stand der Technik bekannt, sie an ihrer Oberfläche zu modifizieren.

So ist aus "The Chemistry of Silica" von Ralph K. Iler (1979, John Wiley & Sons, Inc.; New York, Chichester, Brisbane, Toronto) bekannt, Silikatoberflächen mit Chlorsilanen zu modifizieren.

EP 0 982 268 A beschreibt die Umsetzung wässriger Kieselsole in Anwesenheit wassermischbarer Lösemittel mit Halogensilanen und Mischungen aus Siloxanen und Halogensilanen bzw. Siloxanen.

US 6,736,891 beschreibt die Umsetzung einer wässrigen Suspension von Fällungskieselsäuren bei einem niedrigen pH-Wert mit Hexamethyldisiloxan in Gegenwart von Isopropanol.

Die aus dem Stand der Technik bekannten modifizierten Siliziumdioxid-Partikel bzw. deren Herstellungsverfahren weisen nur eine unzureichende Flexibilität auf. So ist die Anpassung der Partikel für die Weiterverarbeitung zu Kompositen nur eingeschränkt möglich.

Aus der US 2,801,185 ist ein organisches oberflächenmodifiziertes Siliziumdioxid-Partikel bekannt sowie ein Verfahren zu dessen Herstellung, wobei ein Wasser enthaltendes Kieselsol mit einem organischen Lösemittel versetzt und das Wasser azeotrop entfernt wird, so dass der Wasseranteil auf unter 1 % herabgesetzt wird. Anschließend wird ein Modifizierungsmittel (Beschichtungsmaterial) zugegeben und die Modifikation der Oberfläche vorgenommen. Als Beschichtungsmittel werden unter anderem gesättigte primäre und sekundäre Alkohole erwähnt. Diese Partikel haben aber den Nachteil, dass sie nicht die gewünschte Stabilität aufweisen und/oder z.T. nicht die gewünschte Flexibilität in der Weiterverarbeitung erlauben.

US 09/524,907 offenbart ein mehrstufiges Verfahren zur Herstellung oberflächenmodifizierter Siliziumdioxid Partikel durch Umsetzung von wässrigem Kieselsol im ersten Schritt mit einem Alkoxysilan und im zweiten Schritt einem weiteren Modifizierungmittel ausgewählt aus Silazanen oder monofunktionellen Silanen, wobei das Wasser durch Destillation abgetrennt wird und gegen ein organisches Lösemittel getauscht wird.

Aus der US 2,786,042 ist bekannt, organische Kieselsole mit kohlenwasserstoffhaltigen Silanolen an der Oberfläche zu modifizieren.

Nachteilig an diesen bekannten Verfahren ist demnach, dass daraus modifizierte Partikel resultieren, welche nur eine bedingte Redispergierbarkeit aufweisen. Darüber hinaus weisen sie eine nur eingeschränkte Verträglichkeit mit organischen Lösemitteln, wie Toluol und Hexan, oder organischen Harzen und Polymeren, auf. Außerdem lassen sich die Partikel nur in engen Grenzen verändern, so dass eine flexible Anpassung ist nicht möglich.

Die Aufgabe der vorliegenden Erfindung besteht somit in der Bereitstellung oberflächenmodifizierter Kieselsolteilchen (Siliziumdioxid-Partikeln), welche eine verbesserte Redispergierbarkeit oder bessere Verträglichkeit/Komptabilität in bestimmten organischen Lösemitteln zeigen, insbesondere in Toluol.

Gelöst wird diese Aufgabe durch Siliziumdioxid-Partikel, dessen Oberfläche durch einen Belegungsgrad wie folgt modifiziert ist

(a) 0,1 bis 16 Gruppen/nm$^2$ der Art (Modifizierung vom Typ A)

$$(\text{Oberfläche-SiO})_x\text{-Si}(R^1)_y(OR^2)_{4-x-y}$$

mit x = 1 bis 3, y = 1 bis 3 und x + y = 2 oder 3; und

(b) 0,1 bis 16 Gruppen/nm$^2$ der Art (Modifizierung vom Typ B)

$$(\text{Oberfläche-SiO})_z\text{SiR}^3{}_{4-z}$$

mit z = 1 oder 2

aufweist, wobei die Reste $R^1$, $R^2$ und $R^3$ beliebige organische Reste darstellen können und mehrere Reste $R^1$, $R^2$ oder $R^3$ gleich oder verschieden sein können.

Erfindungsgemäß wurde herausgefunden, dass die derart an der Oberfläche modifizierten Partikel eine hervorragende Redispergierbarkeit in organischen Lösemitteln und eine hervorragende Verträglichkeit mit Lösemitteln, wie Toluol, aufweisen. Dieses liegt vorrangig darin begründet, dass ihre Oberfläche durch die zuvor beschriebene Modifizierung mit organischen Resten versehen wird. Wesentlich dabei ist, dass die Partikel tatsächlich die beiden erfindungsgemäß verschiedenen Modifizierungen nach A und B aufweisen. Die Erfindung erfasst demnach keine Varianten, in denen die Modifizierung vom Typ A gleich der Modifizierung vom Typ B ist. Die Modifizierung vom Typ A ist daher ungleich der Modifizierung vom Typ B (A≠B).

Gegenstand der Erfindung sind deshalb oberflächenmodifizierte Siliziumdioxid-Partikel oder Kieselsol, erhältlich durch Umsetzung von wässrigem Kieselsol mit mindestens einem ersten Modifizierungsmittel, umfassend mindestens ein Alkoxysilan, und mindestens einem zweiten Modifizierungsmittel, umfassend Mischungen aus Halogensilan und Siloxan erhalten, wobei Wasser vor der Umsetzung mit dem ersten oder zweiten Modifizierungsmittel entfernt wird.

Die erfindungsgemäßen Siliziumdioxid-Partikel weisen eine Modifizierung der Art (Oberfläche-SiO)$_x$-Si(R)$_y$(OR)$_{4-x-y}$ (Modifizierungsart vom Typ A) von vorzugsweise 0,1 bis 16, vorzugsweise 0,1 bis 10 Gruppen/nm$^2$, insbesondere 0,15 bis 6 Gruppen/nm$^2$, besonders bevorzugt 0,2 bis 4 Gruppen/nm$^2$, auf.

Ferner weisen sie eine Modifizierung der Art (Oberfläche-SiO)$_z$-SiR$^3{}_{4-z}$ (Modifizierungsart vom Typ B) von 0,1 bis 16, vorzugsweise 0,2 bis 10 Gruppen/nm$^2$, insbesondere 0,3 bis 6 Gruppen/nm$^2$, besonders bevorzugt 0,4 bis 4 Gruppen/nm$^2$, auf.

Die vorstehend genannten Vorzugsbereiche für Modifizierungsarten vom Typ A und B können beliebig miteinander kombiniert werden. Die konkrete Kombination hängt von den Notwendigkeiten der weiteren Einsatzbereiche und der Weiterverarbeitung der Partikel ab. Bevorzugt ist eine Kombination von insbesondere 0,9 - 3,6 Gruppen/nm$^2$ der Modifizierung vom Typ A und 0,5 - 3 Gruppen/nm$^2$ der Modifizierung vom Typ B.

Dem Fachmann sind Methoden zur Bestimmung von funktionalen Gruppen auf der Oberfläche der Partikel, und somit auch der Belegung der erfindungsgemäßen Partikel, bekannt. So lassen sich Gruppen aus R$_2$Si und R$_3$Si mit Hilfe von Basen (z.B. Kaliumhydroxyd) abspalten, die dann Disiloxane (R$_3$SiOSiR$_3$) oder Cyclen (R$_2$SiO)n bilden. Diese lassen sich im GC analysieren. Die Methode ist beispielsweise in EP 0982268 B1 erwähnt (Vergleichsbeispiel und Beispiel 1). Auch sind die Gruppen grundsätzlich über NMR und IR bestimmbar. Vinylgruppen lassen sich außerdem über die sog. Iodzahl titrimetrisch erfassen, nämlich durch die Umsetzung der Vinylgruppen mit sog. Wijs-Lösung und anschließender Titration des überschüssigen Halogens mit Natriumthiosulfat. Die Bestimmung von (Meth)acryloylgruppen kann mit Differential Scanning Calorimetry (DSC) bei der Umsetzung mit standardisierten Peroxidlösungen anhand der freiwerdenden Reaktionswärme erfolgen.

## (Modifizierung vom Typ A

Die Modifizierung der Partikeloberfläche vom Typ A erfolgt durch Umsetzung von Kieselsolen mit Alkoxysilanen der allgemeinen Formel (I)

$$\text{Formel (I)} \qquad R^1{}_x\text{Si}(OR^2)_{4-x},$$

in welchen der Rest $R^1$ einem gegebenenfalls substituierten oder funktionalisierten $C_1$-$C_{18}$-Alkylrest entspricht und der Rest $R^2$ ausgewählt sein kann aus der Gruppe, bestehend aus einem gegebenenfalls substituierten oder funktionalisierten $C_1$-$C_{18}$-Alkylrest, einem Carboxyrest, einem gegebenenfalls substituierten $C_2$-$C_{18}$-Alkenylrest und einem Oximrest.

Im Rahmen der vorliegenden Erfindung werden die Verbindungen der allgemeinen Formel (I) vorzugsweise ausgewählt aus der Gruppe, bestehend aus Methyltrimethoxysilan, Trimethylmethoxysilan, Methylhydrogendimethoxysilan, Dimethyldimethoxysilan, Ethyltrimethoxysilan, Ethyltriacetoxysilan, Propyltrimethoxysilan, Diisopropyldimethoxysilan, Diisobutyldimethoxysilan , Chlorpropyltrimethoxysilan, Chlorpropylmethyldimethoxysilan, Chlorisobutylmethyldimethoxysilan, Trifluorpropyltrimethoxysilan, Trifluorpropylmethyldimethoxysilan, iso-Butyltrimethoxysilan, n-Butyltrimethoxysilan, n-Butylmethyldimethoxysilan, Phenyltrimethoxysilan, Phenyltrimethoxysilan, Phenylmethyldimethoxysilan, Triphenylsilanol, n-Hexyltrimethoxysilan, n-Octyltrimethoxysilan, iso-Octyltrimethoxysilan, Decyltrimethoxysilan, Hexadecyltrimethoxysilan, Cyclohexylmethyldimethoxysilan, Cyclohexylethyldimethoxysilan, Dicyclopentyldimethoxysilan, tert.-Butylethyldimethoxysilan, tert.-Butylpropyldimethoxysilan, Dicyclohexyldimethoxysilan, Mercaptopropyltrimethoxysilan, Mercaptopropylmethyldimethoxysilan, Bis(triethoxysilylpropyl)disulfid, Bis(triethoxysilylpropyl)tetrasulfid, Aminopropyltrimethoxysilan, m-Aminophenyltrimethoxysilan, Aminopropylmethyldiethoxysilan, Phenylaminopropyltrimethoxysilan, Aminoethylaminopropyltrimethoxysilan, Aminoethylaminopropylmethyldimethoxysilan, Glycidoxypropyltrimethoxysilan, Glycidoxypropylmethyldimethoxysilan, Epoxycyclohexylethyltrimethoxysilan, $\gamma$-Methacryloxypropyltriacetoxysilan, Vinyltriacetoxysilan, Vinyltrimethoxysilan, Methylvinyldimethoxysilan, Vinyldimethylmethoxysilan, Divinyldimethoxysilan, Vinyltris(2-methoxyethoxy)silan, Hexenyltrimethoxysilan, $\gamma$-Methacryloxypropyltrimethoxysilan, Acryloxypropyltrimethoxysilan, Vinylbenzylethylendiaminpropyltrimethoxysilan, Vinylbenzylethylendiaminpropyltrimethoxysilan-Hydrochlorid, Allylethylendiaminpropyltrimethoxysilan, Allyltrimethoxysilan, Allylmethyldimethoxysilan, Allyldimethylmethoxysilan und Hexenyltrimethoxysilan.

Im Rahmen der vorliegenden Erfindung sind insbesondere Silane der allgemeinen Formel (I-1)

$$\text{Formel (I-1)} \qquad R^1Si(OR^2)_3 \ (x=3)$$

bevorzugt, wobei die Reste $R^1$ und $R^2$ die vorgenannte Bedeutung aufweisen.

## Modifizierung vom Typ B

Die Modifizierung der Partikeloberfläche vom Typ B wird über eine Umsetzung mit einem Halogensilan und einem Siloxan durchgeführt.

Die Halogensilane weisen dabei vorzugsweise die allgemeine Formel (II)

$$\text{Formel (II)} \qquad R^3_aH_bSiX_{4-a-b}$$

auf, worin

jedes $R^3$, unabhängig voneinander, ausgewählt ist aus der Gruppe, bestehend aus Kohlenwasserstoffresten mit 1 bis 18 Kohlenstoffatomen oder organofunktionellen Kohlenwasserstoffresten mit 1 bis 18 Kohlenstoffatomen;
X, unabhängig voneinander, ausgewählt ist aus der Gruppe, bestehend aus Fluor, Chlor, Brom und Iod;
a gleich 0, 1, 2 oder 3 ist;
b gleich 0 oder 1 ist; und
a + b = 1, 2 oder 3 ist.

Bevorzugt sind im Rahmen der vorliegenden Erfindung insbesondere Chlorsilane der allgemeinen Formel (II-1)

$$\text{Formel (II-1)} \qquad R^3_aH_bSiCl_{4-a-b},$$

wobei der Rest $R^3$ und die Indizes a und b die vorgenannte Bedeutung aufweisen.

Besonders bevorzugt sind im Rahmen der vorliegenden Erfindung Halogensilane der allgemeinen Formel (II-2)

$$\text{Formel (II-2)} \qquad R^3_aH_{3-a}SiCl,$$

wobei der Rest $R^3$ und der Index a die vorgenannte Bedeutung aufweisen.

Im Rahmen der vorliegenden Erfindung werden die Verbindungen der allgemeinen Formel (II) vorzugsweise ausgewählt aus der Gruppe, bestehend aus Chlortrimethylsilan, Bromtrimethylsilan, Iodtrimethylsilan, Dichlordimethylsilan, Dichlormethylsilan, Methyltrichlorsilan, Chlordimethylsilan, Trichlorsilan, Ethyltrichlorsilan, Propyltrichlorsilan, Phenyltrichlorsilan, Dichlordiphenylsilan, n-Hexyltrichlorsilan, n-Octyltrichlorsilan, Chlordimethyloctylsilan, Chlordimethyloctadecylsilan, Vinyltrichlorsilan, Dichlormethylvinylsilan, Chlordimethylvinylsilan, Dichlordivinylsilan, $\gamma$-Methacryloxypropyldimethylchlorsilan, Allyltrichlorsilan, Allyldichlormethylsilan und Allylchlordimethylsilan.

Die Siloxane weisen die bevorzugte allgemeine Struktur (III)

$$\text{Formel (III)} \qquad R^3_nSiO_{(4-n)/2}$$

auf, worin

jedes $R^3$, unabhängig voneinander, ausgewählt ist aus der Gruppe, bestehend aus Kohlenwasserstoffresten mit 1 bis 18 Kohlenstoffatomen, organofunktionellen Kohlenwasserstoffresten mit 1 bis 18 Kohlenstoffatomen, einem Wasserstoffatom und einer OH-Gruppe; und n eine Zahl zwischen 2 und einschließlich 3 ist.

Bevorzugt sind im Rahmen der vorliegenden Erfindung Siloxane der allgemeinen Formel (III-1)

Formel (III-1)         $R^3_3SiOSiR^3_3$,

wobei der Rest $R^3$ die vorgenannte Bedeutung aufweist und mehrere $R^3$ jeweils eine unterschiedliche Bedeutung aufweisen können.

Weiter bevorzugt sind im Rahmen der vorliegenden Erfindung cyclische Siloxane der allgemeinen Formel (III-2)

Formel (III-2)         $(R^3_2SiO)_n$,

wobei n eine ganze Zahl ist und mehrere $R^3$ jeweils eine unterschiedliche Bedeutung aufweisen können.

Weiter bevorzugt sind im Rahmen der vorliegenden Erfindung Polysiloxane der allgemeinen Formel (III-3)

Formel (III-3)         $R^3_3SiO(R^3_2SiO)_nSiR^3_3$,

wobei n eine ganze Zahl ist und mehrere $R^3$ jeweils eine unterschiedliche Bedeutung aufweisen können.

In einer bevorzugten Ausführungsform wird ein Polysiloxan der Verbindung III-1 eingesetzt.

Im Rahmen der vorliegenden Erfindung werden die Verbindungen der allgemeinen Formel (III) vorzugsweise ausgewählt aus der Gruppe, bestehend aus Hexamethyldisiloxan, Octamethyltrisiloxan, Hexamethylcyclotrisiloxan, Octamethylcyclotetrasiloxan, Decamethylcyclopentasiloxan, Tetramethyldisiloxan, Trimethylcyclotrisiloxan, Tetramethylcyclotetrasiloxan, Pentamethylcyclopentasiloxan, Divinyltetramethylsiloxan, Trimethyltrivinylcyclosiloxan und Tetramethyltetravinylcyclotetrasiloxan.

## Herstellung der erfindungsgemäßen Partikel bzw. Kieselsole

Die erfindungsgemäßen oberflächenmodifizierten Siliziumdioxid-Partikel oder Kieselsol, werden durch Umsetzung von wässrigem Kieselsol mit mindestens einem ersten Modifizierungsmittel, umfassend mindestens ein Alkoxysilan, und mindestens einem zweiten Modifizierungsmittel, umfassend Mischungen aus Halogensilan und Siloxan erhalten, wobei Wasser vor der Umsetzung mit dem ersten oder zweiten Modifizierungsmittel entfernt wird.

Als Edukt für die erfindungsgemäßen Siliziumdioxid-Partikel kommen Dispersionen von kolloidalem Siliziumdioxid oder Lösungsmitteln in Frage. Dabei kann ein kolloidales Siliziumdioxid verwendet werden, weiches beispielsweise nach der Stöber-Synihese oder aus Wasserglas hergestellt wurde. Unter kolloidalem Siliziumdioxid werden im Sinne der vorliegenden Erfindung Partikel mit einem durchschnittlichen Durchmesser von $\geq$ 1 bis 1000 nm, vorzugsweise von 5 bis 150 nm verstanden. Sie können dispergiert in einer Flüssigkeit vorliegen (Kieselsol). Die Partikel bestehen im Wesentlichen, nämlich vorzugsweise zu mindestens 90%, besonders bevorzugt zu mindestens 95 oder 99% aus Siliziumdioxid.

Bei der Stöber-Synthese werden Alkoxysilane, wie Tetramethoxysilan, in Anwesenheit von Säuren oder Basen als Katalysator hydrolysiert und dadurch gezielt Teilchen aufgebaut. Der Vorteil dieses Verfahrens besteht darin, dass sich sehr enge Teilchengrößenverteilungen und eine beliebige Teilchengröße zwischen 5 und 500 nm erreichen lassen.

Bei der Herstellung von kolloidalem Kieselsol ausgehend von Wasserglas wird eine wässrige Lösung von Natriumsilikat mittels eines Ionenaustauschers entionisiert, wodurch Kieselsäure $(Si(OH)_4)$ gebildet wird. Die entstehende Kieselsäure ist im Allgemeinen instabil und polymerisiert unmittelbar zu kleinen Saat-Partikeln, aus denen dann die eigentlichen Partikel aufgebaut werden. Durch ein geeignetes Einstellen der Prozessbedingungen können enge Partikelgrößenverteilungen im Bereich von beispielsweise etwa 5 bis 150 nm hergestellt werden. Die wässrigen Kieselsole werden im Allgemeinen mit Basen stabilisiert, wodurch die sauren Kieselsäurepartikel negativ geladen werden und sich abstoßen. Wenn im Rahmen der vorliegenden Erfindung ein Kieselsol als Ausgangsmaterial verwendet wird, welches beispielsweise herstellungsbedingt eine Base enthält, wird diese Base jedoch vorzugsweise zunächst entfernt.

Auch kommerziell erhältliche Siliziumdioxid-Partikel, beispielsweise Bindzil 40/130 und Bindzil 40/220 (erhältlich von Eka Chemicals); Levasil 200/40% (erhältlich von H.C. Starck); Nalco 2327, Nalco 1144 und Nalco 2329 (erhältlich von Nalco Company); NexSil 12 und NexSil 20 (erhältlich von Nyacol); Snowtex ST-40 und Snowtex ST-50 (erhältlich von Nissan Chemical American Corporation) können verwendet werden.

Das erfindungsgemäße Verfahren geht somit bevorzugt von einem nanoskaligen, kolloidalen Kieselsäuresol aus. Der pH-Wert dieses Sols wird bevorzugt auf 5 oder weniger, weiter bevorzugt auf 4 oder weniger eingestellt. Bei einem basischen Sol kann dies durch Zugabe von Säure oder durch Verwendung eines sauren Kationenaustauschers geschehen.

Die Umsetzung mit dem ersten und dem zweiten Modifizierungsmittel kann sowohl aufeinanderfolgend als auch gleichzeitig mit einer Mischung aus dem ersten und dem zweiten Modifizierungsmittel erfolgen.

Im Rahmen des erfindungsgemäßen Verfahrens wird aus dem Kieselsol zu einem beliebigen Zeitpunkt des Verfahrensablaufs vorzugsweise Wasser entfernt, da größere Mengen an Wasser im Reaktionsgemisch dazu führen, dass das Reaktionssystem insgesamt polarer ist. Die Entfernung des Wassers kann demnach vor der Umsetzung mit dem ersten oder vor der Umsetzung mit dem zweiten Modifizierungsmittel oder auch zu beiden Zeitpunkten erfolgen. Sofern ein Gemisch an Modifizierungsmitteln eingesetzt wird, kann die Entfernung bereits vor der Umsetzung mit diesem Gemisch erfolgen. Vorzugsweise erfolgt die Entfernung von Wasser vor der Umsetzung mit einem Modifizierungsmittel oder Modifizierungsmittelgemisch enthaltend Halogensilan und/oder ein Organosilan

Im Sinne der Erfindung bedeutet die "Entfernung von Wasser" die Verringerung des Wassergehalts des Systems bezogen auf den Gehalt an Siliziumdioxid auf nicht mehr als 90 Gew.-%, vorzugsweise nicht mehr als 75 Gew.-%, 50 Gew.-%, 35 Gew.-%, 20 Gew.-%, oder 10 Gew.-%. Beachtlich dabei ist, dass der Wassergehalt üblicher, kommerziell erhältlicher Siliziumdioxidsole bei mindestens etwa 50 Gew.-% liegt; meistens jedoch etwa zwischen 60 Gew.-% und 80 Gew.-% (der Wassergehalt bezogen auf das Siliziumdioxid ist in diesen Fällen größer als 100 Gew.-%). Nach einer erfindungsgemäßen Wasserentfernung kann der Gesamtgehalt des Systems an Wasser daher unter 15 Gew.-%, vorzugsweise auch unter 10 Gew.-%, oder unter 7,5 Gew.-% oder unter 5 Gew.-% liegen.

Wenn, wie im vorliegenden erfindungsgemäßen Verfahren vorzugsweise vorgesehen, Wasser aus dem Reaktionssystem entfernt wird, führt dieses zu der Möglichkeit, mit einer einem höheren Gehalt von beispielsweise bis zu 15 Gew.-%, besonders bevorzugt bis 20 Gew.-%, insbesondere bis 25 Gew.-%, des Kieselsols zu arbeiten. Damit lässt sich durch das erfindungsgemäße Verfahren eine deutlich höhere Raum-Zeit-Ausbeute realisieren.

Darüber hinaus ist es bekannt, dass Wasser die Agglomeration von Silika-Teilchen fördert. Deshalb ist es bevorzugt, Wasser aus dem Reaktionssystem des Kieselsols zu entfernen.

In einer Ausführungsform der vorliegenden Erfindung umfasst das erfindungsgemäße Verfahren damit sowohl die Verfahrensschritte

(1) der Umsetzung von kolloidalem Kieselsol mit mindestens einem ersten Modifizierungsmittel, umfassend mindestens ein Alkoxysilan,

(2) der Umsetzung von kolloidalem Kieselsol mit mindestens einem zweiten Modifizierungsmittel, ausgewählt aus einem Halogensilan, einem Siloxan und Mischungen davon, sowie

(3) die Entfernung von Wasser aus dem Kieselsol, insbesondere durch azeotrope Destillation.

Die dabei vorgesehene Reihenfolge der einzelnen Verfahrensschritte (1) bis (3) ist nicht beschränkt und variabel. So ist es im Rahmen der vorliegenden Erfindung grundsätzlich möglich, bei einer zweistufigen Modifizierung der Oberfläche von einem Kieselsol das Wasser vor der ersten Oberflächenmodifizierung oder zwischen der ersten und der zweiten Oberflächenmodifizierung aus dem Reaktionssystem zu entfernen.

In einer bevorzugten Ausführungsform des erfindungsgemäßen Verfahrens erfolgt jedoch die Umsetzung des kolloidalen Kieselsols zunächst mit mindestens einem ersten Modifizierungsmittel, umfassend mindestens ein Alkoxysilan (Verfahrensschritt (1)), vor der Entfernung von Wasser aus dem Kieselsol (3), an welche sich die Umsetzung mit mindestens einem zweiten Modifizierungsmittel, ausgewählt aus einem Halogensilan, einem Siloxan und Mischungen davon (Verfahrensschritt (2)), anschießt.

Im folgenden werden die einzelnen Verfahrensschritte (1) bis (3) näher beschrieben, wobei - wie bereits ausgeführt - die Bezeichnung die Nummerierung der jeweiligen Verfahrensschritte keine Beschränkung der Reihenfolge der Verfahrensschritte bedeutet.

## Verfahrensschritt (1)

Die Modifizierung der Partikeloberfläche gemäß dem Verfahrensschritt (1) kann erfolgen durch die Umsetzung von Kieselsolen mit Alkoxysilanen der allgemeinen Formel (I)

$$\text{Formel (I)} \qquad R^1_x Si(OR^2)_{4-x},$$

in welchen der Rest $R^1$ einem gegebenenfalls substituierten $C_1$-$C_{18}$-Alkylrest entspricht und der Rest $R^2$ ausgewählt sein kann aus der Gruppe, bestehend aus einem gegebenenfalls substituierten $C_1$-$C_{18}$-Alkylrest, einem Carboxyrest, einem gegebenenfalls substituierten $C_2$-$C_{18}$-Alkenylrest und einem Oximrest.

Beachtlich ist, dass durch eine Hydrolyse der entstehenden SiOR Gruppen SiOH Gruppen entstehen können, an denen wiederum Alkyoxysilane anlagern können. Damit können ganz oder teilweise Schichten entstehender, die Si(R)-x-O-Si(R)x-Verknüpfungen aufweisen.

Im Rahmen der vorliegenden Erfindung werden die Verbindungen der allgemeinen Formel (I) vorzugsweise ausgewählt aus der Gruppe, bestehend aus Methyltrimethoxysilan, Trimethylmethoxysilan, Methylhydrogendimethoxysilan, Dimethyldimethoxysilan, Ethyltrimethoxysilan, Ethyltriacetoxysilan, Propyltrimethoxysilan, Diisopropyldimethoxysilan, Diisobutyldimethoxysilan, Chlorpropyltrimethoxysilan, Chlorpropylmethyldimethoxysilan, Chlorisobutylmethyldimethoxysilan, Trifluorpropyltrimethoxysilan, Trifluorpropylmethyldimethoxysilan, iso-Butyltrimethoxysilan, n-Butyltrimethoxysilan, n-Butylmethyldimethoxysilan, Phenyltrimethoxysilan, Phenyltrimethoxysilan, Phenylmethyldimethoxysilan, Triphenylsilanol, n-Hexyltrimethoxysilan, n-Octyltrimethoxysilan, iso-Octyltrimethoxysilan, Decyltrimethoxysilan, Hexadecyltrimethoxysilan, Cyclohexylmethyldimethoxysilan, Cyclohexylethyldimethoxysilan, Dicyclopentyldimethoxysilan, tert.-Butylethyldimethoxysilan, tert.-Butylpropyldimethoxysilan, Dicyclohexyldimethoxysilan, Mercaptopropyltrimethoxysilan, Mercaptopropylmethyldimethoxysilan, Bis(triethoxysilylpropyl)disulfid, Bis(triethoxysilylpropyl)tetrasulfid, Aminopropyltrimethoxysilan, m-Aminophenyltrimethoxysilan, Aminopropylmethyldiethoxysilan, Phenylaminopropyltrimethoxysilan, Aminoethylaminopropyltrimethoxysilan, Aminoethylaminopropylmethyldimethoxysilan, Glycidoxypropyltrimethoxysilan, Glycidoxypropylmethyldimethoxysilan, Epoxycyclohexylethyltrimethoxysilan, $\gamma$-Methacryloxypropyltriacetoxysilan, Vinyltriacetoxysilan, Vinyltrimethoxysilan, Methylvinyldimethoxysilan, Vinyldimethylmethoxysilan, Divinyldimethoxysilan, Vinyltris(2-methoxyethoxy)silan, Hexenyltrimethoxysilan, $\gamma$-Methacroyloxypropyltrimethoxysilan, Acryloxypropyltrimethoxysilan, Vinylbenzylethylendiaminpropyltrimethoxysilan, Vinylbenzylethylendiaminpropyltrimethoxysilan-Hydrochlorid, Allylethylendiaminpropyltrimethoxysilan, Allyltrimethoxysilan, Allylmethyldimethoxysilan, Allyldimethylmethoxysilan und Hexenyltrimethoxysilan.

Im Rahmen der vorliegenden Erfindung sind insbesondere Silane der allgemeinen Formel (I-1)

$$\text{Formel (I-1)} \qquad R^1Si(OR^2)_3 \ (x=3)$$

bevorzugt, wobei die Reste $R^1$ und $R^2$ die vorgenannte Bedeutung aufweisen.

Der Verfahrensschritt (1) wird vorzugsweise bei den folgenden Bedingungen durchgeführt: Für Verfahrensschritt (1) wird das Kieselsol sofern es basisch stabilisiert ist, mit einem Kationentauscher behandelt. Das saure Kieselsol wird dann zur Reaktion gebracht.

Ist das Silan entsprechend Formel (I-1) ausreichend löslich in dem Kieselsol, erfolgt die Reaktion bevorzugt bei Raumtemperatur innerhalb von zwei Stunden. Ist das Silan nicht ausreichend löslich im Kieselsol (erkennbar an Fettaugen nach 15 Minuten intensivem Mischen), muss die Mischung mit einem wassermischbaren Lösungsmittel verdünnt werden. Besonders bevorzugt ist dafür Isopropanol oder 1-Methoxy-2-propanol. Die Mischung mit dem Silan kann auch erwärmt werden.

Die bevorzugte Menge Silan lässt sich anhand der spezifischen Partikeloberfläche $A_0$ berechnen. Es werden bevorzugt 1,5 - 6*$\mu$mol(Silan)/g(SiO$_2$) * $A_0$ eingesetzt. Je größer die Partikel sind desto kleiner ist die spezifische Oberfläche und umso geringer ist die benötigte Menge Silan. Als spezifische Oberfläche kann die Oberfläche nach der BET-Methode oder nach der unten beschriebenen Methode auf Basis der Teilchengröße heran gezogen werden.

Unter diesen Bedingungen reagiert das Silan vollständig mit der Partikeloberfläche, so dass die Beladung mit Gruppen im Wesentlichen der eingesetzten Stöchiometrie entspricht.

## Verfahrensschritt (2)

Die Modifizierung der Kleseisoloberfläche gemäß dem Verfahrensschritt (2) erfolgt beispielsweise durch die Umsetzung des Kieselsols mit einem Halogensilan und einem Siloxan.

Die Halogensilane weisen dabei vorzugsweise die allgemeine Formel (II)

$$\text{Formel (II)} \qquad R^3_aH_bSiX_{4-a-b}$$

auf, worin

jedes $R^3$, unabhängig voneinander, ausgewählt ist aus der Gruppe, bestehend aus Kohlenwasserstoffresten mit 1 bis 18 Kohlenstoffatomen oder organofunktionellen Kohlenwasserstoffresten mit 1 bis 18 Kohlenstoffatomen;
X, unabhängig voneinander, ausgewählt ist aus der Gruppe, bestehend aus Fluor, Chlor, Brom und Iod;
a gleich 0, 1, 2 oder 3 ist;
b gleich 0 oder 1 ist; und
a + b = 1, 2 oder 3 ist.

Die für das erfindungsgemäße Verfahren einsetzbaren Halogensilane weisen vorteilhafterweise die Eignung auf, Partikel mit der Modifizierung des Typ B zu erzeugen. Dazu ist a bevorzugt 1-3, besonders bevorzugt 2 oder 3.

Besonders bevorzugt wird ein Halogensilan, weiter vorzugsweise ein Chlorsilan verwendet. Die Silane können funktionalisiert sein, beispielsweise mit polymerisierbaren Gruppen, insbesondere Vinylgruppen.

Bevorzugt sind im Rahmen der vorliegenden Erfindung Chlorsilane der allgemeinen Formel (II-1)

$$\text{Formel (II-1)} \qquad R^3{}_aH_bSiCl_{4-a-b},$$

wobei der Rest $R^3$ und die Indizes a und b die vorgenannte Bedeutung aufweisen.

Besonders bevorzugt sind im Rahmen der vorliegenden Erfindung Halogensilane der allgemeinen Formel (II-2)

$$\text{Formel (II-2)} \qquad R^3{}_aH_{3-a}SiCl,$$

wobei der Rest $R^3$ und der Index a die vorgenannte Bedeutung aufweisen.

Im Rahmen der vorliegenden Erfindung werden die Verbindungen der allgemeinen Formel (II) vorzugsweise ausgewählt aus der Gruppe, bestehend aus Chlortrimethylsilan, Bromtrimethylsilan, Iodtrimethylsilan, Dichlordimethylsilan, Dichlormethylsilan, Methyltrichlorsilan, Chlordimethylsilan, Trichlorsilan, Ethyltrichlorsilan, Propyltrichlorsilan, Phenyltrichlorsilan, Dichlordiphenylsilan, n-Hexyltrichlorsilan, n-Octyltrichlorsilan, Chlordimethyloctylsilan, Chlordimethyloctadecylsilan, Vinyltrichlorsilan, Dichlormethylvinylsilan, Chlordimethylvinylsilan, Dichlordivinylsilan, γ-Methacryloxypropyldimethylchlorsilan, Allyltrichlorsilan, Allyldichlormethylsilan und Allylchlordimethylsilan.

Die Siloxane weisen die bevorzugte allgemeine Struktur (III)

$$\text{Formel (III)} \qquad R^3{}_nSiO_{(4-n)/2}$$

auf, worin

jedes $R^3$, unabhängig voneinander, ausgewählt ist aus der Gruppe, bestehend aus Kohlenwasserstoffresten mit 1 bis 18 Kohlenstoffatomen, organofunktionellen Kohlenwasserstoffresten mit 1 bis 18 Kohlenstoffatomen, einem Wasserstoffatom und einer OH-Gruppe; und n eine Zahl zwischen 2 und einschließlich 3 ist.

Bevorzugt sind im Rahmen der vorliegenden Erfindung Disiloxane der allgemeinen Formel (III-1)

$$\text{Formel (III-1)} \qquad R^3{}_3SiOSiR^3{}_3,$$

wobei $R^3$ die zuvor angegebene Bedeutung aufweist und mehrere $R^3$ jeweils eine unterschiedliche Bedeutung aufweisen können.

Weiter bevorzugt sind im Rahmen der vorliegenden Erfindung cyclische Siloxane der allgemeinen Formel (III-2)

$$\text{Formel (III-2)} \qquad (R^3{}_2SiO)_n,$$

wobei n eine ganze Zahl ist und mehrere $R^3$ jeweils eine unterschiedliche Bedeutung aufweisen können.

Weiter bevorzugt sind im Rahmen der vorliegenden Erfindung Polysiloxane der allgemeinen Formel (III-3)

$$\text{Formel (III-3)} \qquad R^3{}_3SiO(R^3{}_2SiO)_nSiR^3{}_3,$$

wobei n eine ganze Zahl ist und mehrere $R^3$ jeweils eine unterschiedliche Bedeutung aufweisen können.

Im Rahmen der vorliegenden Erfindung werden die Verbindungen der allgemeinen Formel (III) vorzugsweise ausgewählt aus der Gruppe, bestehend aus Alkyltrimethoxysilane mit 8 oder mehr C-Atomen (z.B. Octyltrimethoxysilan, Iso-Octyltrimethoxysilan, Hexadecyltrimethoxysilan, Octadecyltrimethoxysilan und Methacryloxypropyltrimethoxysilan), Hexamethyldisiloxan, Octamethyltrisiloxan, Hexamethylcyclotrisiloxan, Octanmethylcyclotetrasiloxan, Decamethylcyclopentasiloxan, Tetramethyldisiloxan, Trimethylcyclotrisiloxan, Tetramethylcyclotetrasiloxan, Pentamethylcyclopentasiloxan, Divinyltetramethylsiloxan, Trimethyltrivinylcyclosiloxan und Tetramethyltetravinylcyclotetrasiloxan.

Durch die Menge an dem Modifizierungsmittel in dem zweiten Verfahrensschritt, die Temperatur der Umsetzung und Reaktionsdauer der Umsetzung lassen sich die Eigenschaften der resultierenden Kleselsole, wie die Polarität und die Redispergierbarkeit, steuern.

Der Verfahrensschritt (2) wird vorzugsweise bei den folgenden Bedingungen durchgeführt:

Die Reaktion wird mit einer Mischung aus Chlorsilanen und Siloxanen durchgeführt. Bei den Siloxanen sind insbesondere die Disiloxane bevorzugt, da überschüssige Disiloxane nach der Reaktion aus dem Gemisch über eine Destillation abgetrennt werden können.

Die Reaktion wird bevorzugt im organischen Medium bei einem Wassergehalt zwischen 1 und 10 % durchgeführt. Die bevorzugte Reaktionstemperatur liegt wenig unter der Siedetemperatur des Gemisches. Beim Einsatz von 1 mmol (Chlorsilan)/ g($SiO_2$) kann bei 70 °C innerhalb von 2 Stunden eine vollständige Reaktion erreicht werden.

Je nach Menge Siloxan und Halogensilan wird ein Belegungsgrad von bis 90 % der SiOH-Gruppen auf der Oberfläche erreicht.

Kolloidales Silica hat üblicherweise ca. 4,6 SiOH-Gruppen pro $nm^2$.

## Verfahrensschritt (3)

Die Entfernung von Wasser aus dem Kieselsol kann beispielsweise durch eine Extraktion mit einer Phasentrennung, eine Destillation, eine azeotrope Destillation oder durch ein Membranverfahren erfolgen.

In einer bevorzugten Ausführungsform des erfindungsgemäßen Verfahrens erfolgt die Entfernung von Wasser durch eine azeotrope Destillation mit einem organischen Lösemittel. Die azeotrope Destillation bietet den Vorteil, dass das Wasser aus dem Kieselsolsystem entfernt werden kann, ohne dass dabei auf die Eignung des organischen Lösemittels zur Phasentrennung zu achten wäre. Da eine azeotrope Destillation im Allgemeinen mit fast allen organischen Lösemitteln, mit welchen Wasser ein Azeotrop bildet, gelingt, erhöht sich durch die azeotrope Destillation die Auswahlmöglichkeiten für das organische Lösemittel, so dass insgesamt ein Verfahren mit größerer Flexibilität resultiert.

Das organische Lösemittel, welches zur azeotropen Entfernung von Wasser aus dem Kieselsol verwendet wird, unterliegt keiner besonderen Beschränkung und es kann jedes beliebige Lösemittel verwendet wird, welches mit Wasser ein Azeotrop bildet. Bevorzugt ist dabei die Verwendung eines Lösemittels, welches zu einem wassermischbaren System aus Kieselsol und Lösemittel führt. Bevorzugt sind daher Lösemittel, welche sich mit Wasser im Wesentlichen vollständig, auch unter Verwendung von oberflächenaktiven Mitteln, mischen lassen.

Geeignete Lösemittel für die azeotrope Destillation können beispielsweise ausgewählt werden aus der Gruppe, bestehend aus Alkoholen, wie Methanol, Ethanol, n-Propanol, Isopropanol, Pentanole, Octanole und Cyclohexanol; Glycole, wie Ethylenglykol und Diethylenglykol; Ether, Glycol- und Propylenglykolether, wie Diethylether, Dibutylether, Anisol, 1,4-Dioxan, 1,3-Dioxan, 1,3-Dioxolan, Tetrahydrofuran, 1-Methoxy-2-propanol, 1-Methoxy-1-propanol, 2-Methoxyethanol, 1-Ethoxy-2-propanol, Mono-, Di-, Tri- und Polyethylenglycolether; Ketone und Aldehyde, wie Aceton, Butanon und Cyclohexanon; Ester, wie Essigsäureester und Glycolester; Amide und andere stickstoffhaltige Lösemittel, wie Dimethylformamid und Nitrobenzol, Piperidin, N-Methylpiperidin und Acetonitril; schwefelhaltige Lösemittel, wie Dimethylsulfoxid; Halogenkohlenwasserstoffe, wie Dichlormethan, Chloroform, Tetrachlormethan, Tri- und Tetrachlorethan, 1,2-Dichlorethan, Chlorfluorkohlenwasserstoffe; Kohlenwasserstoffe, wie Pentan, Hexan, Heptan, Benzine, Petrolether, Cyclohexan, Methylcyclohexan, Decalin, Terpene, Benzol, Toluol und Xylole; und dergleichen. Insbesondere bevorzugt ist Isopropanol.

Das erfindungsgemäße Verfahren kann darüber hinaus weitere optionale Verfahrensschritte, beispielsweise das Entfernen von flüchtigen Bestandteilen, wie von überschüssigen Silanen, umfassen, was vorzugsweise durch eine Destillation erfolgt.

In einer weiteren bevorzugten Ausführungsform der vorliegenden Erfindung wird die Modifizierung der Kieselsole in einem sauren pH-Wert durchgeführt, wobei es jedoch nicht möglich ist, den genauen sauren pH-Wert näher zu konkretisieren, da die erfindungsgemäße Umsetzung in einem organischen Lösemittel durchgeführt wird.

Die vorliegende Erfindung betrifft darüber hinaus die nach dem oben beschriebenen Verfahren erhältlichen Kieselsole.

Ein weiterer Gegenstand der vorliegenden Erfindung ist die Verwendung der erfindungsgemäßen Kieselsole bzw. der nach dem oben beschriebenen Verfahren erhältlichen Kieselsole. Die erfindungsgemäße Dispersion oder das aus der Dispersion durch Entfernen des Lösemittels gewonnene redispergierbare Pulver können in verschiedenste Basispolymere eingearbeitet werden und deren physikalische und insbesondere mechanische Eigenschaften verbessern bzw. verändern. Als Basispolymere können im Rahmen der Erfindung eine Vielzahl bekannter Polymere verwendet werden. Beispielsweise können mittels der erfindungsgemäßen Systeme thermoplastische oder duroplastische Kunststoffe modifiziert werden. Beispielhaft erwähnt seien Polyolefine, Polycarbonate, Polyamide, Polyimide, Polyacrylate, Polymethacrylate, Polyetherketone, Polysulfone, Polyurethane, Polyharnstoffe, Epoxidharze und Polyesterharze.. Modifizierbare Elastomere sind beispielsweise Naturkautschuk, Butylkautschuke, Acrylatkautschuke, Styrol-Butadien-Kautschuk (SBR), ggf. hydrierte Nitril-Butadien-Kautschuke, Polysiloxane (Silikone) etc. Bei vielen dieser Stoffgruppen ist es von besonderem Vorteil, die erfindungsgemäßen Nanopartikel als redispergierbares Pulver einzuarbeiten, da ein Einbringen über Lösungsmittel nachteilig und mit hohem Aufwand verbunden ist.

Besonders vorteilhaft kann das erfindungsgemäße nanoskalige Siliciumdioxid auch in Polymere bzw. Harze mit niedrigem Siedepunkt eingearbeitet werden, wie beispielsweise Methylmethacrylat (MMA).

Erfindungsgemäß hergestellte Partikel können ebenfalls zur Modifikation von Weichmachern wie beispielsweise Adipaten und Phthalaten verwendet werden. Sie bilden mit diesen Weichmachern niedrigviskose und stabile Dispersionen.

Die erfindungsgemäß hergestellten Partikeln enthaltende polymere bzw. polymerisierbare Mischungen stellen stabile und daher lagerfähige Dispersionen dar und weisen gute Fließeigenschaften (niedrige Viskosität, geringe Strukturviskosität) auf. Sie eignen sich daher beispielsweise für die Herstellung von Dentalformulierungen, die beispielsweise aus einem statischen Mischer appliziert werden und daher keine zu hohe Verarbeitungsviskositäten aufweisen dürfen. Besonders bevorzugt können sie bei Dentalformulierungen auf Basis von Silikonen verwendet werden. Ein anderes mögliches Anwendungsgebiet ist die Modifikation von LSRs (Liquid Silicone Rubber), die in der Regel im Spritzguss verarbeitet werden und bei denen deswegen eine niedrige Verarbeitungsviskosität von großem Vorteil ist. Erfindungsgemäß kann bei LSRs ein hoher Füllstoffgehalt und damit gute mechanische Eigenschaften des ausgehärteten Endprodukts erreicht werden, ohne dass die Verarbeitbarkeit durch eine zu hohe Viskosität darunter leidet.

Grundsätzlich ermöglicht es die Erfindung, polymerisierbare Mischungen bereitzustellen, die aufgrund ihrer niedrigen Viskosität gut verarbeitbar sind und als ausgehärtetes Polymer durch einen hohen Füllstoffgehalt bewirkte verbesserte Eigenschaften aufweisen, insbesondere mechanische Eigenschaften, verbesserte thermische Leitfähigkeit und dergleichen.

Aus den so erhaltenen Kieselsolen sowie den zuvor beschriebenen Kieselsolen lassen sich nach Entfernen des Lösemittels Pulver gewinnen, die in verschiedenen Medien redispergierbar sind. Dabei hat sich überraschenderweise herausgestellt, dass die Partikelgrößenverteilung nach der Dispergierung im wesentlichen der Partikelgrößenverteilung im Lösemittel entspricht, obwohl die Partikel bei der Trocknung agglomerieren, da das stabilisierende Medium wegfällt. Die Agglomeration ist jedoch im vorliegenden erfindungsgemäßen Fall im Wesentlichen reversibel, so dass die Partikel mit einem geringen Energieaufwand wieder in eine Dispersion überführt werden können. Die Trocknung des oberflächenmodifizierten Kieselsols kann zum Beispiel durch die Sprühtrocknung erfolgen.

Der erfindungsgemäße Gegenstand weist eine Reihe an Vorteilen auf. So können durch die Variation des Alkoxysilane bei der ersten Modifizierung die Eigenschaften der Partikel unabhängig von den Halogensilanen und den Siloxanen der zweiten Modifizierung eingestellt werden. Durch die Menge an Halogensilan bzw. Siloxan bei der zweiten Modifizierung kann wiederum die Polarität der resultierenden Kieselsolteilchen beeinflusst werden, da Kieselsolteilchen, welche nur mit einem Alkoxysilan beschichtet werden, im Allgemeinen relativ polar sind, während durch die zweite Modifizierung die Beschichtung insgesamt wieder unpolarer wird. Durch eine geschickte Kombination der Art und Menge der ersten und zweiten Modifizierung lassen sich Partikel herstellen, die in vorgegebenen Lösemitteln maßgeschneidert eine stabile Dispersion ergeben. Das erfindungsgemäße Verfahren erlaubt gewissermaßen eine Baukastenchemie zum gezielten Einstellen von Polarität und gleichzeitig eine Abschirmung der Oberfläche.

Da es sich bei der zweiten Modifizierung um eine Gleichgewichtsreaktion handelt, kann der Anteil an unpolaren Silylgruppen auf der Oberfläche über die Menge an Silan bei der Reaktion gezielt eingestellt werden.

Die erfindungsgemäßen bzw. nach dem erfindungsgemäßen Verfahren erhältlichen Kieselsolen können zur Herstellung redispergierbarer Pulver verwendet werden.

Durch die Verwendung der erfindungsgemäßen Kieselsole lassen sich die mechanischen Eigenschaften, insbesondere die Zugfestigkeit, das E-Modul, die Weiterreißfestigkeit, das Biegemodul und die Schlagzähigkeit, in Elastomeren, Verbundwerkstoffen und thermoplastischen Werkstoffen verbessern. Bei der Verwendung der erfindungsgemäßen Kieselsole in der Herstellung z.B. von optischen Linsen lassen sich höhere Brechungsindizes erreichen. Auch die Gasbarriereeigenschaften, das Brandverhalten sowie die Fließeigenschaften werden durch die erfindungsgemäßen Kieselsoldispersionen verbessert. Darüber hinaus können die erhaltenen oberflächenmodifizierten Kieselsole in Dispersionsform beispielsweise zur Herstellung von Verbundstoffen (Nanocomposite) verwendet werden. Demnach sind ein weiterer Gegenstand der Erfindung die mit den erfindungsgemäßen Kieselsolen erhältlichen Verbundstoffe (Nanocomposites). Diese sind aufgrund ihrer verbesserten mechanischen Eigenschaften, z.B. erhöhte Kratz- und Abriebfestigkeit (Tribologie), vorteilhaft. Dies gilt beispielsweise für die Verwendung in Lacken.

Die vorliegende Erfindung wird durch die folgenden Beispiele näher beschrieben, welche die vorliegende Erfindung jedoch nicht beschränken.

### Beispiele:

### Methode zur Teilchengrößenbestimmung

Die Teilchengröße kann in Lösung mittels dynamischer Licht-streuung (DLS) auf einem "Dynamic Light Scattering Particle Size Analyzer LB-550" der Firma Horiba bei einer Konzentration von maximal 10 Gew.-% Partikeln erfolgen, wobei die Dispersion dazu maximal eine dynamische Viskosität von 3 mPas bei 25 °C aufweisen sollte. Als Teilchengröße wird der Median (D50-Wert) der Partikelgrößenverteilung angeben.

Im Feststoff kann die Teilchengröße durch Transmissionselektronenmikroskopie bestimmt werden. Dazu werden mindestens 100 Partikel ausgemessen und eine Partikelgrößenverteilung gebildet.

**Bestimmung der Oberfläche**

Die Oberfläche wird auf Basis der Partikelgröße berechnet. Dabei wird davon ausgegangen, dass die Partikel alle den gleichen Durchmesser entsprechend des Medians ($d_{50}$-Wert) der Partikelgrößenverteilung und eine sphärische Form aufweisen.

Für die spezifische Oberfläche ($nm^2$/g (Partikel)) gilt:

$$A_0 = 6 \; / \; (\rho \; x \; d_{50}),$$

wobei $\rho$ die Dichte der Partikel ist (Dichte ($SiO_2$) = 2,1 $g/cm^3$). Die Zahl der Gruppen N ergibt sich aus:

$$N = ([Mol \; (reaktive \; Gruppen] \; / \; [Masse \; der \; Partikel]) \; x \; 6{,}022 \; x \; 10^{23}$$

Der Quotient ($N \; / \; A_0$) ergibt die Zahl der Gruppen pro Oberflächeneinheit.

Bei der Reaktion von Alkoxysilanen kann vereinfachend angenommen werden, dass das eingesetzte Silan vollständig auf der Oberfläche der Partikel hydrolysiert.

**Vergleichsbeispiele**

Ein basisches kolloidales Kieselsol (40 Gew.-% $SiO_2$ in Wasser; mittlere Teilchengröße $d_{50}$ (bestimmt mittels dynamischer Lichtstreuung): 25 nm; stabilisiert mit NaOH) wurde über einen sauren Ionenaustauscher (Amberjet 1200H, erhältlich von Rohm & Haas) gerührt, bis ein pH-Wert von 2 bis 3 erreicht war. Nachdem der Ionenaustauscher durch Filtration entfernt war, wurde das saure Sol mit verschiedenen Alkoxysilanen (siehe Ziffern 2 bis 5 der Tabellen 1 und 2 unten) 2 h lang gerührt. Das Beispiel 1 wurde ohne Alkoxysilan durchgeführt und diente daher zum Vergleich.

Das Sol wurde anschließend mit Isopropanol verdünnt und unter Zugabe von weiterem Isopropanol wurde das Gemisch von Lösemittel und Wasser bei reduziertem Druck abdestilliert. Das erhaltene Sol wurde unter Rühren mit Chlortrimethylsilan und Hexamethyldisiloxan versetzt. Das Gemisch wurde zwei Stunden bei 70 °C gerührt, durch Zugabe von Amberjet 4400 OH neutralisiert und der Ionenaustauscher abfiltriert

**Ergebnisse**

Zum Vergleich der Eigenschaften der Partikel mit unterschiedlichen Alkoxysilanen (siehe Ziffern 2 bis 5 in den Tabellen 1 und 2) in der ersten Beschichtung wurden Kieselsole bei 40 °C im Vakuum getrocknet. Die resultierenden Pulver wurden in Toluol redispergiert, so dass Sole mit 10 Gew.-% Feststoffanteil entstanden. Diese wurden über dynamische Lichtstreuung vermessen.

**Tabelle 1**

| Beispiel | Teilchengröße $d_{50}$ [nm] | Spanne $(d_{90}\text{-}d_{10})/(d_{50})$ | Viskosität [mPas] |
|---|---|---|---|
| 1) Kein Alkoxysilan | 696 | 4,0 | 20 |
| 2) Propyltrimethoxysilan | 30,3 | 0,7 | 0,9 |
| 3) Octyltrimethoxysilan | 28,0 | 0,7 | 0,7 |

Die Auswertung der Tabelle 1 zeigt, dass je näher die gemessene Partikelgröße an der ursprünglich vorhandenen Partikelgröße und -verteilung liegt, desto besser sind die Partikel geeignet, in Toluol redispergiert zu werden. An den Ergebnissen in Tabelle 1 lässt sich erkennen, dass das im ersten Verfahrensschritt eingesetzte Alkoxysilan die Redispergierbarkeit der Partikel in Toluol erheblich verbessert. Auch die Viskosität der Dispersionen ist ein Maß für die Verträglichkeit der Partikel zu der Matrix (Lösemittel). Die mit Alkoxysilan umgesetzten Partikel verursachen in Toluol eine erheblich niedrigere Viskosität als die Partikel ohne Alkoxysilan, d.h. sie sind mit Toluol besser verträglich.

Werden unterschiedliche Kieselsole über Lösemittelaustausch in Toluol überführt und mittels DLS vermessen, kann man die Polarität der Partikel anhand eines Vergleichs mit dem ursprünglichen Isopropanolsol ablesen.

**Tabelle 2**

| Alkoxysilan | Teilchengröße $d_{50}$ in Isopropanol [nm] | Teilchengröße $d_{50}$ in Toluol [nm] |
|---|---|---|
| 4) Phenyltrimethoxysilan | 104 | 46 |
| 5) $\gamma$-Methacryloxypropyltri methoxysilan | 44 | 4470 |

Die Verträglichkeit von Partikeln mit Lösemitteln lässt sich an der Partikelgröße in der dynamischen Lichtstreuung ablesen. Partikel, die mit dem Lösemittel nicht verträglich sind, lagern sich zusammen und erscheinen in der dynamischen Lichtstreuung bei größerer Teilchengröße. Je näher die gemessenen Teilchengrößen an der tatsächlichen Teilchengröße liegt, desto weniger Partikel sind also zusammengelagert.

Die Ergebnisse aus Tabelle 2 machen deutlich, dass die Partikel, die mit dem $\gamma$-Methacryloxypropyltrimethoxysilan umgesetzt wurden, mit dem polareren Lösemittel Isopropanol besser verträglich sind, als mit dem unpolareren Toluol. Die polare $\gamma$-Methacryloxypropyl-Gruppe ermöglicht eine gute Verträglichkeit mit unpolaren Lösemitteln. Diese Ergebnisse zeigen, dass die vorliegende Erfindung im Sinne einer "Baukastenchemie" zum gezielten Anbringen gewünschter Moleküle mittels Silylierung dienen kann.

**Vorschrift für die Beispiele 1 - 3 (Tabelle 1)**

Ein basisches kolloidales Kieselsol (40 Gew.-% $SiO_2$ in Wasser, Teilchengröße (DLS) D50 = 25 nm, stabilisiert mit NaOH) wurde mit saurem Ionentauscher Amberjet 1200 H (Rohm & Haas) gerührt, bis ein pH-Wert von 2 erreicht war. 100 Teile des Sols wurden mit 0,24 mmol des Alkoxysilans / Teil(Sol) für 2 h gerührt (außer bei Beispiel 1). Dann wurde mit 600 Teilen Isopropanol verdünnt und das Sol bei 40 - 50 °C im Vakuum auf ca. 150 Teile eingeengt.

Das Sol wurde durch Zugabe von Isopropanol auf 300 Gewichtsteile aufgefüllt und dann mit einem Gemisch aus 4,4 Teilen Chlortrimethylsilan und 13 Teilen Hexamethyldisiloxan versetzt und 2 Stunden bei 70 °C gerührt. Nach dem Entfernen der Heizung wurden 25 Teile Amberjet 4400 OH (basischer Ionentauscher, Rohm & Haas) dazu geben. Nach einer weiteren Stunde Rühren wurde der Ionentauscher abfiltriert.

**Vorschrift für die Beispiele 4 und 5 (Tabelle 2)**

Ein basisches kolloidales Kieselsol (40 Gew.-% $SiO_2$ in Wasser, Teilchengröße (DLS) D50 = 25 nm, Stabilisiert mit NaOH) wurde mit saurem Ionentauscher Amberjet 1200 H (Rohm & Haas) gerührt, bis ein pH-Wert von 2 erreicht war. 100 Teile des Sols wurden mit 0,24 mmol des Alkoxysilans / Teil(Sol) für 2 h gerührt. Dann wurde mit 600 Teilen Isopropanol verdünnt und das Sol bei 40 - 50 °C im Vakuum auf ca. 150 Teile eingeengt.

Das Sol wurde durch Zugabe von Isopropanol auf 160 Gewichtsteile aufgefüllt. Dann wurde mit einem Gemisch aus 4,4 Teilen Chlortrimethylsilan und 13 Teilen Hexamethyldisiloxan versetzt und 2 Stunden bei 70 °C gerührt. Nach dem Entfernen der Heizung wurden 25 Teile Amberjet 4400 OH (basischer Ionentauscher, Rohm & Haas) dazu geben. Nach einer weiteren Stunde Rühren wurde der Ionentauscher abfiltriert.

**Beispiel 6 (Tabelle 3)**

Ein basisches kolloidales Kieselsol (40 Gew.-% $SiO_2$ in Wasser, Teilchengröße (DLS) D50 = 25 nm, stabilisiert mit NaOH) wurde mit saurem Ionentauscher Amberjet 1200 H (Rohm & Haas) gerührt, bis ein pH-Wert von 2 erreicht war. 100 Teile des Sols wurden mit 50 Teilen Isopropanol verdünnt und mit einem Gemisch aus 0,24 mmol Propyltrimethoxysilan / Teil(Sol), 4,4 Teilen Chlortrimethylsilan und 13 Teilen Hexamethyldisiloxan versetzt und 2 Stunden bei 70 °C gerührt. Nach dem Entfernen der Heizung wurden 25 Teile Amberjet 4400 OH (basischer Ionentauscher, Rohm & Haas) dazu geben. Nach einer weiteren Stunde Rühren wurde der Ionentauscher abfiltriert. Dann wurde mit 550 Teilen Isopropanol verdünnt und das Sol bei 40 - 50 °C im Vakuum auf ca. 150 Teile eingeengt.

**Beispiel 7 (Tabelle 3)**

Ein basisches kolloidales Kieselsol (40 Gew.-% $SiO_2$ in Wasser, Teilchengröße (DLS) D50 = 25 nm, stabilisiert mit NaOH) wurde mit saurem Ionentauscher Amberjet 1200 H (Rohm & Haas) gerührt, bis ein pH-Wert von 2 erreicht war. 100 Teile des Sols wurden mit 200 Teilen Isopropanol verdünnt und mit einem Gemisch aus 0,24 mmol Propyltrimethoxysilan / Teil(Sol), 4,4 Teilen Chlortrimethylsilan und 13 Teilen Hexamethyldisiloxan versetzt und 2 Stunden bei 70 °C gerührt. Nach dem Entfernen der Heizung wurden 25 Teile Amberjet 4400 OH (basischer Ionentauscher, Rohm & Haas) dazu geben. Nach einer weiteren Stunde Rühren wurde der Ionentauscher abfiltriert. Dann wurde mit 400 Teilen Isopropanol verdünnt und das Sol bei 40 - 50 °C im Vakuum auf ca. 150 Teile eingeengt.

**Beispiel 8 (Tabelle 3)**

Ein basisches kolloidales Kieselsol (40 Gew.-% $SiO_2$ in Wasser, Teilchengröße (DLS) D50 = 25 nm, stabilisiert mit NaOH) wurde mit saurem Ionentauscher Amberjet 1200 H (Rohm & Haas) gerührt, bis ein pH-Wert von 2 erreicht war. 100 Teile des Sols wurden mit 600 Teilen Isopropanol verdünnt und das Sol bei 40 - 50 °C im Vakuum auf ca. 150 Teile eingeengt.

Das Sol wurde mit einem Gemisch aus 0,24 mmol Propyltrimethoxysilan / Teil(Sol), 4,4 Teilen Chlortrimethylsilan und 13 Teilen Hexamethyldisiloxan versetzt und 2 Stunden bei 70 °C gerührt. Nach dem Entfernen der Heizung wurden 25 Teile Amberjet 4400 OH (basischer Ionentauscher, Rohm & Haas) dazu geben. Nach einer weiteren Stunde Rühren wurde der Ionentauscher abfiltriert.

**Vergleich der Beispiele 6 bis 8**

Die Kieselsole aus den Beispielen 6 bis 8 wurden bei 40 °C im Vakuum getrocknet und dann in Toluol redispergiert, so dass Sole mit 10 Gew.-% entstanden.

| Beispiel | Teilchengröße $d_{50}$ [nm] | Spanne $(d_{90}-d_{10})/d_{50}$ | Viskosität [mPas] |
|---|---|---|---|
| 6 | 3130 | 2,1 | 1,9 |
| 7 | 4220 | 1,1 | 2,8 |
| 8 | 91,9 | 1,8 | > 20 |

**Patentansprüche**

1. Oberflächenmodifizierte Siliziumdioxid-Partikel oder Kieselsol, erhältlich durch Umsetzung von wässrigem Kieselsol mit mindestens einem ersten Modifizierungsmittel, umfassend mindestens ein Alkoxysilan, und mindestens einem zweiten Modifizierungsmittel, umfassend Mischungen aus Halogensilan und Siloxan, wobei Wasser vor der Umsetzung mit dem ersten oder zweiten Modifizierungsmittel entfernt wird.

2. Oberflächenmodifizierte Siliziumdioxid-Partikel nach Anspruch 1, **dadurch gekennzeichnet, dass** die Umsetzungen mit dem ersten und zweiten Modifizierungsmittel entweder aufeinanderfolgend oder gleichzeitig mit einer Mischung aus mindestens einem ersten und mindestens einem zweiten Modifizierungsmittel erfolgt.

3. Oberflächenmodifizierte Siliziumdioxid-Partikel oder Kieselsol nach Anspruch 1 oder 2, **dadurch gekennzeichnet, dass** das Wasser vor der ersten Oberflächenmodifizierung oder zwischen der ersten und der zweiten Oberflächenmodifizierung, vorzugsweise durch azeotrope Destillation, aus dem Reaktionssystem entfernt wird.

4. Oberflächenmodifizierte Siliziumdioxid-Partikel oder Kieselsol nach einem der vorherigen Ansprüche, **dadurch gekennzeichnet, dass** der Wassergehalt des Kieselsols durch die Entfernung des Wassers bezogen auf den Gehalt an Siliziumdioxid bei nicht mehr als 90 Gew.-%, 75 Gew.-%, 50 Gew.-%, 35 Gew.-%, 20 Gew.-% oder 10 Gew.-% liegt bzw. der Gesamtgehalt des Systems an Wasser unter 15 Gew.-%, vorzugsweise unter 10 Gew.-%, unter 7,5 Gew.-% oder unter 5 Gew.-% liegt.

5. Oberflächenmodifizierte Siliziumdioxid-Partikel oder Kieselsol nach einem der vorherigen Ansprüche, **dadurch gekennzeichnet, dass** das Alkoxysilan der allgemeinen Formel (I) $R^1_x Si(OR^2)_{4-x}$ entspricht, in welchen der Rest $R^1$ einem gegebenenfalls substituierten oder funktionalisierten $C_1$-$C_{18}$-Alkylrest entspricht und der Rest $R^2$ ausgewählt sein kann aus der Gruppe, bestehend aus einem gegebenenfalls substituierten oder funktionalisierten $C_1$-$C_{18}$-Alkylrest, einem Carboxyrest, einem gegebenenfalls substituierten $C_2$-$C_{18}$-Alkenylrest und einem Oximrest, oder bevorzugt der allgemeinen Formel (I-1) $R^1 Si(OR^2)_3$ entspricht, in welcher der Rest $R^1$ einem gegebenenfalls substituierten oder funktionalisierten $C_1$-$C_{18}$-Alkylrest entspricht und der Rest $R^2$ ausgewählt ist aus der Gruppe, bestehend aus einem gegebenenfalls substituierten oder funktionalisierten $C_1$ - $C_{18}$-Alkylrest, einem Carboxyrest, einem gegebenenfalls substituierten $C_2$ - $C_{18}$-Alkenylrest und einem Oximrest, besonders bevorzugt sind Silane der Formel (I-1) mit $R^2$ = Methyl oder Ethyl.

6. Oberflächenmodifizierte Siliziumdioxid-Partikel oder Kieselsol nach einem der vorherigen Ansprüche, **dadurch gekennzeichnet, dass** das Alkoxysilan ausgewählt ist aus den Alkoxysilanen der folgenden Gruppe:

Methyltrimethoxysilan, Trimethylmethoxysilan, Methylhydrogendimethoxysilan, Dimethyldimethoxysilan, Ethyltrimethoxysilan, Ethyltriacetoxysilan, Propyltrimethoxysilan, Diisopropyldimethoxysilan, Diisobutyldimethoxysilan, Chlorpropyltrimethoxysilan, Chlorpropylmethyldimethoxysilan, Chlorisobutylmethyldimethoxysilan, Trifluorpropyltrimethoxysilan, Trifluorpropylmethyldimethoxysilan, iso-Butyltrimethoxysilan, n-Butyltrimethoxysilan, n-Butylmethyldimethoxysilan, Phenyltrimethoxysilan, Phenyltrimethoxysilan, Phenylmethyldimethoxysilan, Triphenylsilanol, n-Hexyltrimethoxysilan, n-Octyltrimethoxysilan, iso-Octyltrimethoxysilan, Decyltrimethoxysilan, Hexadecyltrimethoxysilan, Cyclohexylmethyldimethoxysilan, Cyclohexylethyldimethoxysilan, Dicyclopentyldimethoxysilan, tert.-Butylethyldimethoxysilan, tert.-Butylpropyldimethoxysilan, Dicyclohexyldimethoxysilan, Mercaptopropyltrimethoxysilan, Mercaptopropylmethyldimethoxysilan, Bis(triethoxysilylpropyl)disulfid, Bis(triethoxysilylpropyl)tetrasulfid, Aminopropyltrimethoxysilan, m-Aminophenyltrimethoxysilan, Aminopropylmethyldiethoxysilan, Phenylaminopropyltrimethoxysilan, Aminoethylaminopropyltrimethoxysilan, Aminoethylaminopropylmethyldimethoxysilan, Glycidoxypropyltrimethoxysilan, Glycidoxypropylmethyldimethoxysilan, Epoxycyclohexylethyltrimethoxysilan, γ-Methacryloxypropyltriacetoxysilan, Vinyltriacetoxysilan, Vinyltrimethoxysilan, Methylvinyldimethoxysilan, Vinyldimethylmethoxysilan, Divinyldimethoxysilan, Vinyltris(2-methoxyethoxy)silan, Hexenyltrimethoxysilan, γ-Methacroyloxypropyltrimethoxysilan, Acryloxypropyltrimethoxysilan, Vinylbenzylethylendiaminpropyltrimethoxysilan, Vinylbenzylethylendiaminpropyltrimethoxysilan-Hydrochlorid, Allylethylendiaminpropyltrimethoxysilan, Allyltrimethoxysilan, Allylmethyldimethoxysilan, Allyldimethylmethoxysilan und Hexenyltrimethoxysilan.

7. Oberflächenmodifizierte Siliziumdioxid-Partikel oder Kieselsol nach einem der vorhergehenden Ansprüche, **dadurch gekennzeichnet, dass** das Halogensilan der allgemeinen Formel (II) $R^3{}_aH_bSiX_{4-a-b}$ entspricht, in welcher jedes $R^3$, unabhängig voneinander, ausgewählt ist aus der Gruppe, bestehend aus Kohlenwasserstoffresten mit 1 bis 18 Kohlenstoffatomen oder organofunktionellen Kohlenwasserstoffresten mit 1 bis 18 Kohlenstoffatomen;
X, unabhängig voneinander, ausgewählt ist aus der Gruppe, bestehend aus Fluor, Chlor, Brom und Iod;
a gleich 0, 1, 2 oder 3 ist;
b gleich 0 oder 1 ist; und
a+b gleich 1, 2 oder 3 ist,
bevorzugt sind $R^3{}_aH_bSiCl_{4-a-b}$, insbesondere bevorzugt sind $R^3{}_aH_{3-a}SiCl$.

8. Oberflächenmodifizierte Siliziumdioxid-Partikel oder Kieselsol nach einem der vorhergehenden Ansprüche, **dadurch gekennzeichnet, dass** das Siloxan der allgemeinen Formel (III) $R^3{}_nSiO_{(4-n)/2}$ entspricht, in welcher jedes $R^3$, unabhängig voneinander, ausgewählt ist aus der Gruppe, bestehend aus Kohlenwasserstoffresten mit 1 bis 18 Kohlenstoffatomen oder organofunktionellen Kohlenwasserstoffresten mit 1 bis 18 Kohlenstoffatomen, einem Wasserstoffatom und einer OH-Gruppe; und n eine Zahl zwischen 2 und einschließlich 3 ist, bevorzugt sind Siloxane der allgemeinen Formeln $R^3{}_3SiOSiR^3{}_3$, $(R^3{}_2SiO)n$ oder $R^3{}_3SiO(R^3{}_2SiO)_nSiR^3{}_3$, besonders bevorzugt sind Siloxane der Formel $R^3SiOSiR^3$, wobei n eine ganze Zahl ist und mehrere $R^3$ jeweils eine unterschiedliche Bedeutung aufweisen können.

9. Oberflächenmodifizierte Siliziumdioxid-Partikel oder Kieselsol nach einem der vorhergehenden Ansprüche, **dadurch gekennzeichnet, dass** das Alkoxysilan ausgewählt ist aus Propyltrimethoxysilan, Phenyltrimethoxysilan, Alkyltrimethoxysilane mit 8 oder mehr C-Atomen (z.B. Octyltrimethoxysilan, Iso-Octyltrimethoxysilan, Hexadecyltrimethoxysilan, Octadecyltrimethoxysilan) und Methacryloxypropyltrimethoxysilan und das zweite Modifizierungsmittel aus einer Mischung aus Chlortrimethylsilan und Hexamethyldisiloxan besteht.

10. Oberflächenmodifizierte Siliziumdioxid-Partikel oder Kieselsol nach einem der vorhergehenden Ansprüche, **dadurch gekennzeichnet, dass** es in getrockneter Form vorliegt.

11. Oberflächenmodifizierte Siliziumdioxid-Partikel oder Kieselsol nach einem der vorhergehenden Ansprüche, **dadurch gekennzeichnet, dass** es nach dem Trocknen redispergierbar ist.

12. Oberflächenmodifizierte Siliziumdioxid-Partikel oder Kieselsol nach Anspruch 11, **dadurch gekennzeichnet, dass** die Partikelgrößenverteilung nach der Dispergierung im Wesentlichen der Partikelgrößenverteilung im Lösemittel entspricht.

13. Verwendung eines oberflächenmodifizierten Siliziumdioxid-Partikels oder Kieselsols gemäß einem der vorhergehenden Ansprüche in Basispolymeren, beispielsweise Olefinen, Polycarbonaten, Polyamiden, Polyimiden, Polyacrylaten, Polymethacrylaten, Polyetherketonen, Polysulfonen, Polyurethanen, Polyharnstoffen, Epoxidharzen, Polyesterharzen, Polysiloxanen, Naturkautschuk, Butylkautschuken, Acrylatkautschuken, Styrol-Butadien-Kautschuken (SBR), gegebenenfalls hydrierten Nitril-Butadien-Kautschuken und Methylmethacrylat (MMA).

14. Polymere, bzw. polymerisierbare Mischungen, enthaltend oberflächenmodifizierte Siliziumdioxid-Partikel oder Kieselsol gemäss einem der Ansprüche 1 bis 13.

15. Verwendung von Polymeren gemäß Anspruch 14 für die Herstellung von Dentalformulierungen.

16. Verwendung von Polymeren gemäß Anspruch 15 für die Modifikation von LSRs (Liquide Silicone Rubber).

17. Verfahren zur Herstellung oberflächenmodifizierter Siliziumdioxid-Partikel oder Kleselsol, durch Umsetzung von wässrigem Kleselsol mit mindestens einem ersten Modifizierungsmittel, umfassend mindestens ein Alkoxysilan, und mindestens einem zweiten Modifizierungsmittel, umfassend Halogensilan oder Mischungen aus Halogensilan und Siloxan wobei Wasser vor der Umsetzung mit dem ersten oder zweiten Modifizierungsmittel entfernt wird.

**Claims**

1. Surface-modified silicon dioxide particles or silica sol obtainable by reacting aqueous silica sol with at least one first modifier comprising at least one alkoxysilane and with at least one second modifier comprising mixtures of halosilane and siloxane, with water being removed prior to reaction with the first or second modifier.

2. Surface-modified silicon dioxide particles according to Claim 1, **characterized in that** the reactions with the first and second modifiers takes place either successively or simultaneously with a mixture of at least one first and at least one second modifier.

3. Surface-modified silicon dioxide particles or silica sol according to Claim 1 or 2, **characterized in that** the water is removed before the first surface modification or between the first and second surface modifications, preferably by azeotropic distillation, from the reaction system.

4. Surface-modified silicon dioxide particles or silica sol according to any of the preceding claims, **characterized in that** the water content of the silica sol as a result of the removal of the water, based on the silicon dioxide content, is not more than 90%, 75%, 50%, 35%, 20% or 10% by weight, and/or the total water content of the system is below 15%, preferably below 10%, below 7.5% or below 5% by weight.

5. Surface-modified silicon dioxide particles or silica sol according to any of the preceding claims, **characterized in that** the alkoxysilane is of the general formula (I) $R^1_x Si(OR^2)_{4-x}$ in which the radical $R^1$ is an optionally substituted or functionalized $C_1$-$C_{18}$ alkyl radical and radical $R^2$ may be selected from the group consisting of an optionally substituted or functionalized $C_1$-$C_{18}$ alkyl radical, a carboxyl radical, an optionally substituted $C_2$-$C_{18}$ alkenyl radical, and an oxime radical, or preferably is of the general formula (I-1) $R^1 Si(OR^2)$ in which the radical $R^1$ is an optionally substituted or functionalized $C_1$-$C_{18}$ alkyl radical and the radical $R^2$ is selected from the group consisting of an optionally substituted or functionalized $C_1$-$C_{18}$ alkyl radical, a carboxyl radical, an optionally substituted $C_2$-$C_{18}$ alkenyl radical, and an oxime radical, more preferably silanes of the formula (I-1) with $R^2$ = methyl or ethyl.

6. Surface-modified silicon dioxide particles or silica sol of any of the preceding claims, **characterized in that** the alkoxysilane is selected from the alkoxysilanes of the following group:

methyltrimethoxysilane, trimethylmethoxysilane, methylhydrodimethoxysilane, dimethyldimethoxysilane, ethyltrimethoxysilane, ethyltriacetoxysilane, propyltrimethoxysilane, diisopropyldimethoxysilane, diisobutyldimethoxysilane, chloropropyltrimethoxysilane, chloropropylmethyldimethoxysilane, chloroisobutyl-methyldimethoxysilane, trifluoropropyltrimethoxysilane, trifluoropropylmethyldimethoxysilane, isobutyltrimethoxysilane, n-butyltrimethoxysilane, n-butylmethyldimethoxysilane, phenyltrimethoxysilane, phenyltrimethoxysilane, phenylmethyldimethoxysilane, triphenylsilanol, n-hexyltrimethoxysilane, n-octyltrimethoxysilane, isooctyltrimethoxysilane, decyltrimethoxysilane, hexadecyltrimethoxysilane, cyclohexylmethyldimethoxysilane, cyclohexylethyldimethoxysilane, dicyclopentyldimethoxysilane, tert-butylethyldimethoxysilane, tert-butylpropyldimethoxysilane, dicyclohexyldimethoxysilane, mercaptopropyltrimethoxysilane, mercaptopropylmethyldimethoxysilane, bis(triethoxysilylpropyl) disulfide, bis(triethoxysilylpropyl) tetrasulfide, aminopropyltrimethoxysilane, m-aminophenyltrimethoxysilane, aminopropylmethyldiethoxysilane, phenylaminopropyl-trimethoxysilane, aminoethylaminopropyltrimethoxysilane, aminoethylaminopropylmethyldimethoxysilane, glycidyloxypropyltri-

methoxysilane, glycidyloxypropylmethyldimethoxysilane, epoxycyclohexylethyltrimethoxysilane, γ-methacry-loyloxypropyltriacetoxysilane, vinyltriacetoxysilane, vinyltrimethoxysilane, methylvinyldimethoxysilane, vinyld-imethylmethoxysilane, divinyldimethoxysilane, vinyltris(2-methoxyethoxy)silane, hexenyltrimethoxysilane, γ-methacryloyloxypropyltrimethoxysilane, acryloyloxypropyltrimethoxysilane, vinylbenzylethylenediaminopro-pyltrimethoxysilane, vinylbenzylethylenediaminopropyltrimethoxysilane hydrochloride, allylethylenediamine-propyltrimethoxysilane, allyltrimethoxysilane, allylmethyldimethoxysilane, allyldimethylmethoxysilane, and hexenyltrimethoxysilane.

7. Surface-modified silicon dioxide particles or silica sol according to any of the preceding claims, **characterized in that** the halosilane is of the general formula (II) $R^3_aH_bSiX_{4-a-b}$ in which each $R^3$, independently of any other, is selected from the group consisting of hydrocarbon radicals having 1 to 18 carbon atoms or organofunctional hydrocarbon radicals having 1 to 18 carbon atoms;
X, independently at each occurrence, is selected from the group consisting of fluorine, chlorine, bromine, and iodine;
a is 0, 1, 2 or 3;
b is 0 or 1; and
a+b is 1, 2 or 3,
preferably $R^3_aH_bSiCl_{4-a-b}$, more preferably $R^3_aH_{3-a}SiCl$.

8. Surface-modified silicon dioxide particles or silica sol according to any of the preceding claims, **characterized in that** the siloxane is of the general formula (III) $R^3_nSiO_{(4-n)/2}$ in which each $R^3$, independently of any other, is selected from the group consisting of hydrocarbon radicals having 1 to 18 carbon atoms or organofunctional hydrocarbon atoms having 1 to 18 carbon atoms, a hydrogen atom, and an OH group; and n is a number between 2 and 3 inclusive, preferably siloxanes of the generally formulae $R^3_3SiOSiR^3_3$, $(R^32SiO)_n$, or $R^3_3SiO(R^32SiO)_nSiR^3_3$, more preferably siloxanes of the formula $R^3SiOSiR^3$, where n is an integer and two or more radicals $R^3$ may each have a different definition.

9. Surface-modified silicon dioxide particles or silica sol according to any of the preceding claims, **characterized in that** the alkoxysilane is selected from propyltrimethoxysilane, phenyltrimethoxysilane, alkyltrimethoxysilanes having 8 or more C atoms (e.g., octyltrimethoxysilane, isooctyltrimethoxysilane, hexadecyltrimethoxysilane, octadecyltrimethoxysilane), and methacryloyloxypropyltrimethoxysilane, and the second modifier is composed of a mixture of chlorotrimethylsilane and hexamethyldisiloxane.

10. Surface-modified silicon dioxide particles or silica sol according to any of the preceding claims, **characterized in that** it is present in dried form.

11. Surface-modified silicon dioxide particle or silica sol according to any of the preceding claims, **characterized in that** it is redispersible after drying.

12. Surface-modified silicon dioxide particles or silica sol according to Claim 11, **characterized in that** the particle size distribution after dispersion corresponds substantially to the particle size distribution in the solvent.

13. The use of a surface-modified silicon dioxide particle or silica sol as claimed in any of the preceding claims in base polymers, for example, olefins, polycarbonates, polyamides, polyimides, polyacrylates, polymethacrylates, polyetherketones, polysulfones, polyurethanes, polyureas, epoxy resins, polyester resins, polysiloxanes, natural rubber, butyl rubbers, acrylate rubbers, styrene-butadiene rubbers (SBR), optionally hydrogenated nitrile-butadiene rubbers, and methyl methacrylate (MMA).

14. Polymers or polymerizable mixtures comprising surface-modified silicon dioxide particles or silica sol according to any of Claims 1 to 13.

15. Use of polymers according to Claim 14 for producing dental formulations.

16. Use of polymers according to Claim 15 for modifying LSRs (liquid silicone rubber).

17. Process for preparing surface-modified silicon dioxide particles or silica sol by reacting aqueous silica sol with at least one first modifier comprising at least one alkoxysilane and with at least one second modifier comprising halosilane or mixtures of halosilane and siloxane, with water being removed prior to reaction with the first or second modifier.

**Revendications**

1. Particules de dioxyde de silicium modifiées en surface ou sol de silice, pouvant être obtenus par mise en réaction d'un sol de silice aqueux avec au moins un premier agent de modification, comprenant au moins un alcoxysilane, et au moins un deuxième agent de modification, comprenant des mélanges d'halogénosilane et de siloxane, de l'eau étant éliminée avant la mise en réaction avec le premier ou le deuxième agent de modification.

2. Particules de dioxyde de silicium modifiées en surface selon la revendication 1, **caractérisées en ce que** les réactions avec le premier et le deuxième agent de modification ont lieu successivement ou simultanément avec un mélange d'au moins un premier et d'au moins un deuxième agent de modification.

3. Particules de dioxyde de silicium modifiées en surface ou sol de silice selon la revendication 1 ou 2, **caractérisés en ce que** l'eau est éliminée du système réactionnel avant la première modification de surface ou entre la première et la deuxième modification de surface, de préférence par distillation azéotropique.

4. Particules de dioxyde de silicium modifiées en surface ou sol de silice selon l'une quelconque des revendications précédentes, **caractérisés en ce que** la teneur en eau du sol de silice par l'élimination de l'eau est, par rapport à la teneur en dioxyde de silicium, inférieure ou égale à 90 % en poids, 75 % en poids, 50 % en poids, 35 % en poids, 20 % en poids ou 10 % en poids, ou la teneur totale du système en eau est inférieure à 15 % en poids, de préférence inférieure à 10 % en poids, inférieure à 7,5 % en poids ou inférieure à 5 % en poids.

5. Particules de dioxyde de silicium modifiées en surface ou sol de silice selon l'une quelconque des revendications précédentes, **caractérisés en ce que** l'alcoxysilane correspond à la formule générale (I) $R^1_x Si(OR^2)_{4-x}$, dans laquelle le radical $R^1$ correspond à un radical alkyle en $C_1$-$C_{18}$ éventuellement substitué ou fonctionnalisé, et le radical $R^2$ peut être choisi dans le groupe constitué par un radical alkyle en $C_1$-$C_{18}$ éventuellement substitué ou fonctionnalisé, un radical carboxy, un radical alcényle en $C_2$-$C_{18}$ éventuellement substitué et un radical oxime, ou correspond de préférence à la formule générale (1-1) $R^1 Si(OR^2)_3$, dans laquelle le radical $R^1$ correspond à un radical alkyle en $C_1$-$C_{18}$ éventuellement substitué ou fonctionnalisé, et le radical $R^2$ peut être choisi dans le groupe constitué par un radical alkyle en $C_1$-$C_{18}$ éventuellement substitué ou fonctionnalisé, un radical carboxy, un radical alcényle en $C_2$-$C_{18}$ éventuellement substitué et un radical oxime, les silanes de formule (1-1) avec $R^2$ = méthyle ou éthyle étant particulièrement préférés.

6. Particules de dioxyde de silicium modifiées en surface ou sol de silice selon l'une quelconque des revendications précédentes, **caractérisés en ce que** l'alcoxysilane est choisi parmi les alcoxysilanes du groupe suivant :

méthyltriméthoxysilane, triméthylméthoxysilane, méthylhydrogénodiméthoxysilane, diméthyldiméthoxysilane, éthyltriméthoxysilane, éthyltriacétoxysilane, propyltriméthoxysilane, diisopropyldiméthoxysilane, diisobutyldiméthoxysilane, chloropropyltriméthoxysilane, chloropropylméthyldiméthoxysilane, chloroisobutylméthyldiméthoxysilane, trifluoropropyltriméthoxysilane, trifluoropropylméthyldiméthoxysilane, iso-butyltriméthoxysilane, n-butyltriméthoxysilane, n-butylméthyldiméthoxysilane, phényltriméthoxysilane, phényltriméthoxysilane, phénylméthyldiméthoxysilane, triphénylsilanol, n-hexyltriméthoxysilane, n-octyltriméthoxysilane, iso-octyltriméthoxysilane, décyltriméthoxysilane, hexadécyltriméthoxysilane, cyclohexylméthyldiméthoxysilane, cyclohexyléthyldiméthoxysilane, dicyclopentyldiméthoxysilane, tert.-butyléthyldiméthoxysilane, tert.-butylpropyldiméthoxysilane, dicyclohexyldiméthoxysilane, mercaptopropyltriméthoxysilane, mercaptopropylméthyldiméthoxysilane, disulfure de bis(triéthoxysilylpropyle), tétrasulfure de bis(triéthoxysilylpropyle), aminopropyltriméthoxysilane, m-aminophényltriméthoxysilane, aminopropylméthyldiéthoxysilane, phénylaminopropyltriméthoxysilane, aminoéthylaminopropyltriméthoxysilane, aminoéthylaminopropylméthyldiméthoxysilane, glycidoxypropyltriméthoxysilane, glycidoxypropylméthyldiméthoxysilane, époxycyclohexyléthyltriméthoxysilane, $\gamma$-méthacryloxypropyltriacétoxysilane, vinyltriacétoxysilane, vinyltriméthoxysilane, méthylvinyldiméthoxysilane, vinyldiméthylméthoxysilane, divinyldiméthoxysilane, vinyltris(2-méthoxyéthoxy)silane, hexényltriméthoxysilane, $\gamma$-méthacroyloxypropyltriméthoxysilane, acryloxypropyltriméthoxysilane, vinylbenzyléthylènediaminopropyltriméthoxysilane, chlorhydrate de vinylbenzyléthylènediaminopropyltriméthoxysilane, allyléthylènediaminopropyltriméthoxysilane, allyltriméthoxysilane, allylméthyldiméthoxysilane, allyldiméthylméthoxysilane et hexényltriméthoxysilane.

7. Particules de dioxyde de silicium modifiées en surface ou sol de silice selon l'une quelconque des revendications précédentes, **caractérisés en ce que** l'halogénosilane correspond à la formule générale (II) $R^3_a H_b SiX_{4-a-b}$, dans laquelle les $R^3$ sont chacun choisis, indépendamment les uns des autres, dans le groupe constitué par les radicaux hydrocarbonés de 1 à 18 atomes de carbone ou les radicaux hydrocarbonés organofonctionnels de 1 à 18 atomes de carbone ;

les X sont choisis, indépendamment les uns des autres, dans le groupe constitué par fluor, chlore, brome et iode ;

a représente 0, 1, 2 ou 3 ;

b représente 0 ou 1 ; et

a+b représente 1, 2 ou 3,

$R^3_a H_b SiCl_{4-a-b}$ étant préféré, $R^3_a H_{3-a} SiCl$ étant particulièrement préféré.

8. Particules de dioxyde de silicium modifiées en surface ou sol de silice selon l'une quelconque des revendications précédentes, **caractérisés en ce que** le siloxane correspond à la formule générale (III) $R^3_n SiO_{(4-n)/2}$, dans laquelle les $R^3$ sont chacun choisis, indépendamment les uns des autres, dans le groupe constitué par les radicaux hydrocarbonés de 1 à 18 atomes de carbone ou les radicaux hydrocarbonés organofonctionnels de 1 à 18 atomes de carbone, un atome d'hydrogène et un groupe OH ; et n est un nombre compris entre 2 et 3 inclus, les siloxanes de formule générale $R^3_3 SiOSiR^3_3$, $(R^3_2 SiO)_n$ ou $R^3_3 SiO(R^3_2 SiO)_n SiR^3_3$ étant préférés, les siloxanes de formule $R^3 SiOSiR^3$ étant particulièrement préférés, n étant un nombre entier et plusieurs $R^3$ pouvant chacun avoir une signification différente.

9. Particules de dioxyde de silicium modifiées en surface ou sol de silice selon l'une quelconque des revendications précédentes, **caractérisés en ce que** l'alcoxysilane est choisi parmi le propyltriméthoxysilane, le phényltriméthoxysilane, les alkyltriméthoxysilanes contenant 8 atomes C ou plus (p. ex. octyltriméthoxysilane, iso-octyltriméthoxysilane, hexadécyltriméthoxysilane, octadécyltriméthoxysilane) et le méthacryloxypropyltriméthoxysilane, et le deuxième agent de modification est constitué par un mélange de chlorotriméthylsilane et d'hexaméthyldisiloxane.

10. Particules de dioxyde de silicium modifiées en surface ou sol de silice selon l'une quelconque des revendications précédentes, **caractérisés en ce qu'**ils se présentent sous forme sèche.

11. Particules de dioxyde de silicium modifiées en surface ou sol de silice selon l'une quelconque des revendications précédentes, **caractérisés en ce qu'**ils sont redispersibles après le séchage.

12. Particules de dioxyde de silicium modifiées en surface ou sol de silice selon la revendication 11, **caractérisés en ce que** la distribution des tailles de particules après la dispersion correspond essentiellement à la distribution des tailles de particules dans le solvant.

13. Utilisation de particules de dioxyde de silicium modifiées en surface ou d'un sol de silice selon l'une quelconque des revendications précédentes dans des polymères de base, par exemple des oléfines, des polycarbonates, des polyamides, des polyimides, des polyacrylates, des polyméthacrylates, des polyéther-cétones, des polysulfones, des polyuréthanes, des polyurées, des résines époxyde, des résines de polyester, des polysiloxanes, du caoutchouc naturel, des caoutchoucs de butyle, des caoutchoucs d'acrylate, des caoutchoucs de styrène-butadiène (SBR), des caoutchoucs de nitrile-butadiène éventuellement hydrogénés et du méthacrylate de méthyle (MMA).

14. Polymères, ou mélanges polymérisables, contenant des particules de dioxyde de silicium modifiées en surface ou un sol de silice selon l'une quelconque des revendications 1 à 13.

15. Utilisation de polymères selon la revendication 14 pour la fabrication de formulations dentaires.

16. Utilisation de polymères selon la revendication 15 pour la modification de LSR (Liquid Silicone Rubber).

17. Procédé de fabrication de particules de dioxyde de silicium modifiées en surface ou d'un sol de silice, par mise en réaction d'un sol de silice aqueux avec au moins un premier agent de modification, comprenant au moins un alcoxysilane, et au moins un deuxième agent de modification, comprenant un halogénosilane ou des mélanges d'halogénosilane et de siloxane, de l'eau étant éliminée avant la mise en réaction avec le premier ou le deuxième agent de modification.

## IN DER BESCHREIBUNG AUFGEFÜHRTE DOKUMENTE

**In der Beschreibung aufgeführte Patentdokumente**

- EP 0982268 A
- US 6736891 B
- US 2801185 A
- US 09524907 B
- US 2786042 A

**In der Beschreibung aufgeführte Nicht-Patentliteratur**

- **RALPH K. ILER.** The Chemistry of Silica. John Wiley & Sons, Inc, 1979